# EUROPEAN PATENT SPECIFICATION

(11) **EP 1 956 373 B1**
(45) Date of publication and mention of the grant of the patent: **25.01.2017**
(21) Application number: 06834228.6
(22) Date of filing: 07.12.2006
(51) Int. Cl.: G01N 33/531, G01N 33/545, G01N 33/571, G01N 33/92

(54) **METHOD FOR ASSAYING ANTI-TREPONEMA PALLIDUM ANTIBODY**
VERFHAREN ZUM NACHWEIS EINES ANTIKÖRPERS GEGEN TREPONEMA PALLIDUM
PROCÉDÉ POUR LE DOSAGE D'ANTICORPS ANTI-TRÉPONÉMA PALLIDUM

(30) Priority: 07.12.2005 JP 2005354080; 07.12.2005 JP 2005354081
(43) Date of publication of application: 13.08.2008
(73) Proprietor: Sekisui Medical Co., Ltd., Tokyo 103-0027 (JP); NOF Corporation, Ebisu 4-chome, Shibuya-ku Tokyo 150-6019 (JP)
(72) Inventor: AKAMINE, Takayuki, Osaka 618-8589 (JP); OTA, Tetsuya, Osaka 618-8589 (JP); LI, Yan, Osaka 618-8589 (JP)
(74) Representative: Hart-Davis, Jason
(86) International application number: PCT/JP2006/324473
(87) International publication number: WO 2007/066731

(56) References cited:
- WO-A-91/10138
- WO-A-2004/040311
- JP-A- 02 156 157
- JP-A- 11 118 800
- JP-A- 54 151 097
- JP-A- 2001 228 156
- JP-A- 2001 242 171
- JP-A- 2003 337 134
- JP-A- 2003 344 413
- JP-A- 2003 501 662
- GOKAY O ET AL: "Synthesis of diagnostic test kits for syphilis with polymeric particles" JOURNAL OF BIOMATERIALS SCIENCE POLYMER EDITION, vol. 16, no. 5, May 2005 (2005-05), pages 597-610, XP009110342 ISSN: 0920-5063

## Description

### TECHNICAL FIELD

The present invention relates to assaying anti-Treponema pallidum antibodies, which enables the accurate diagnosis of syphilitic infection by preventing an occurrence of serum interference.

### BACKGROUND ART

Infection of Treponema Pallidum, a pathogen of syphilis, in a living body causes a production of a phospholipid-reactive antibody, as well as an antibody against the pathogen, in the living body. A reagent for assaying anti-phospholipid antibodies is a reagent for diagnosing infection of syphilis by assaying the presence or absence of the phospholipid-reactive antibody in blood.

Conventionally, the anti-phospholipid antibody has been assayed by a hand method, such as an RPR card test using a test slide. However, in recent years, a reagent (reagent for an autoanalyzer) applicable to a biochemical autoanalyzer has been commercially available.

In the case that the reagent for an autoanalyzer is used, an amount of serum used in the assay tends to be smaller, compared to an amount thereof used in the assay by the hand method, in order to reduce a load of blood collection on a patient. Accordingly, an amount of antigen-antibody reaction caused by a reaction between the reagent and the antibody in the blood is also small. Therefore, in the case that the reagent for an autoanalyzer is used, a sensitizer may be added to accelerate the antigen-antibody reaction. Examples of the generally-used sensitizer include polyethylene glycol, dextran, and the like. Further, as the sensitizer used with the reagent for assaying anti-phospholipid antibodies, Patent Document 1 discloses that polyvinylpyrrolidone and pullulan effectively work.

However, the sensitizer of this kind has a problem in stability in the case that the reagent is stored for a long time, though it is excellent in accelerating the antigen-antibody reaction, and therefore, the reagent fails to keep its performance due to desensitization of the sensitizer after a long-time storage thereof.

On the other hand, infection of Treponema Pallidum, a pathogen of syphilis, in a living body causes a production of an antibody against the pathogen in the living body. A reagent for assaying anti-Treponema pallidum antibodies is a reagent for diagnosing infection of syphilis by assaying the presence or absence of the anti-Treponema pallidum antibodies in the blood.

Conventionally, the anti-Treponema pallidum antibody has been assayed by a hand method such as TPHA, which utilizes hemagglutination. However, in recent years, a reagent (reagent for an autoanalyzer) applicable to a biochemical autoanalyzer has been commercially available. In the case that the reagent is used, an amount of serum used in the assay tends to be smaller, compared to an amount thereof used in the assay by the hand method, in order to reduce a load of blood collection on a patient. Accordingly, an amount of antigen-antibody reaction caused by a reaction between the reagent and the antibody in the blood is also small. Therefore, in the case that the reagent for an autoanalyzer is used, a sensitizer may be added to accelerate the antigen-antibody reaction. Examples of the generally-used sensitizer include polyethylene glycol, dextran, and the like. Further, as the sensitizer used with the reagent for assaying anti-Treponema pallidum antibodies, Patent Document 2 discloses that a soluble polymer containing a glucoside derivative in a monomer unit and/or a soluble copolymer effectively works.

However, the sensitizer of this kind has a problem that an accurate result of the assay cannot be obtained in some samples, though it is excellent in accelerating the antigen-antibody reaction. More specifically, there is a problem that, in the case that the sample is serum, a phenomenon called serum interference, which negatively affects the assay, may occur due to variation of components contained in the serum, and as a result, the accurate result of the assay cannot be obtained.
Patent Document 1: Japanese Kokai Publication Hei-10-282096
Patent Document 2: Japanese Patent No. 2947600

WO 91/10138 A, GOKAY O ET AL: "Synthesis of diagnostic test kits for syphilis with polymeric particles", JOURNAL OF BIOMATERIALS SCIENCE POLYMER EDITION, vol. 16, no. 5, May 2005 (2005-05), pages 597-610, ISSN: 0920-5063, JP 2003 344413 A and JP 2001 242171 A disclose reagents obtained by immobilisation of phospholipid antigens onto polymers derived from 2-methacryloyloxyehtyl phosphatidyl choline. Specifically JP 2003 344413 A shows this concept for a sensitivity enhancement agent for immune chromatography measuring methods containing polymer which has a basis expressed in a side chain which uses a syphilis related antibody as a measuring object material.

### DISCLOSURE OF THE INVENTION

### PROBLEMS TO BE SOLVED BY THE INVENTION

A purpose of the present invention is, in light of the above-mentioned present situation, assaying for anti-Treponema pallidum antibodies, to enable the accurate diagnosis of syphilitic infection by preventing an occurrence of serum interference.

The invention is defined in the claims.

### MEANS

A reagent for assaying anti-phospholipid antibodies is a reagent for assaying anti-phospholipid antibodies used for diagnosing syphilitic infection, which contains an insoluble carrier supporting a phospholipid antigen thereon and a copolymer having a segment derived from 2-methacryloyloxyethyl phosphorylcholine and having a segment derived from a hydrophilic monomer.

Further, a reagent for assaying anti-Treponema pallidum antibodies is a reagent for assaying anti-Treponema pallidum antibodies used for diagnosing syphilitic infection, which contains an insoluble carrier supporting a Treponema pallidum antigen thereon and a copolymer having a segment derived from 2-methacryloyloxyethyl phosphorylcholine and having a segment derived from a hydrophilic monomer.

In the following, the reagent and the present disclosure will be described in detail.

Inventors of the present invention have extensively conducted research efforts, and as a result, the inventors have found that, by having the reagent for assaying anti-phospholipid antibodies contain a copolymer having a segment derived from 2-methacryloyloxyethyl phosphorylcholine and having a segment derived from a hydrophilic monomer as a sensitizer, it becomes possible to increase assaying sensitivity and to maintain storage stability for a long time.

A copolymer having a segment derived from 2-methacryloyloxyethyl phosphorylcholine and having a segment derived from a hydrophilic monomer is obtained by copolymerizing 2-methoacryloyloxyethyl phosphorylcholine and the hydrophilic monomer (hereinafter and in the claims, also referred to simply as copolymer).

The following general formula (1) indicates a structure of the copolymer in the case that methacrylic acid is used as the hydrophilic monomer.

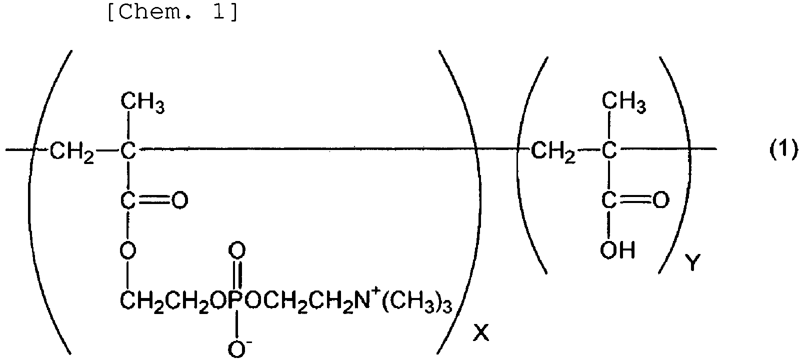

The 2-methacryloyloxyethyl phosphorylcholine is, since a methacryloyl group is included, capable of copolymerizing with another polymerizable monomer. Examples of the copolymerizable hydrophilic monomer include a (meth)acrylic acid monomer, such as 2-hydroxyethyl methacrylate, (meth)acrylate, (meth)acrylamide, and the like.

The hydrophilic monomer is not particularly limited, and examples thereof include (meth)acrylic acid, (meth)acrylamide, and the like. Out of these, (meth)acrylic acid, which is cationic, is suitable, because (meth)acrylic acid is expected to repulse electrostatically from protein and the like in components in the blood.

Here, (meth)acrylic acid indicates acrylic acid or methacrylic acid.

A ratio between the segment derived from 2-methacryloyloxyethyl phosphorylcholine and the segment derived from a hydrophilic monomer in the copolymer is not particularly limited, and can be selected properly as needed. However, it is desirable to be 5: 5 to 3:7 in a molar ratio. In the case that the ratio of the segment derived from 2-methacryloyloxyethyl phosphorylcholine to the segment derived from a hydrophilic monomer is less than this range, an occurrence of serum interference during the assay of the anti-Treponema pallidum antibody may not be prevented efficiently.

A weight-average molecular weight of the copolymer is not particularly limited, however, the desirable lower limit is 5000 and the desirable upper limit is 5 million. In the case that the weight-average molecular weight is less than 5000, an effect of accelerating agglutination may be lost. In contrast, in the case that the weight-average molecular weight is more than 5 million, reproducibility and the like may be deteriorated since viscosity of the reagent becomes too high in the case of adding the copolymer to the reagent.

With regard to a content of the copolymer in the reagent for assaying anti-phospholipid antibodies, the desirable lower limit is 0.1 (w/v) % and the desirable upper limit is 1.2 (w/v) %. In the case that the content is less than 0.1 (w/v)%, the occurrence of serum interference may not be prevented efficiently. Further, in the case that the content is more than 1.2 (w/v)%, the reproducibility may be deteriorated since the viscosity of the reagent becomes too high.

The more desirable lower limit is 0.2 (w/v)% and the more desirable upper limit is 0.8 (w/v)%.

The reagent for assaying anti-phospholipid antibodies contains an insoluble carrier which is a particle supporting a phospholipid antigen thereon.

As the phospholipid antigen, for example, a lipid antigen including cardiolipin, phosphatidylcholine and cholesterol is desirable.

As the cardiolipin, it is desirable to use cardiolipin purified from a bovine heart, however, chemically synthesized cardiolipin may also be used.

As the phosphatidylcholine, it is desirable to use phosphatidylcholine purified from a hen egg yolk, however, lecithin which has a content of phosphatidylcholine of 60 to 80% may also be used.

As the cholesterol, cholesterol of animal origin may be used, or alternatively, chemically-synthesized cholesterol may be used.

A mixing ratio of the cardiolipin, the phosphatidylcholine and the cholesterol is not particularly limited as long as the resulting phospholipid antigen enables determination of the presence or absence of infection of syphilis. However, the ratio is desirable to be 8 to 12 of the phosphatidylcholine and 1 to 5 of the cholesterol, with respect to 1 of the cardiolipin.

It is desirable to use an insoluble carrier including a polymer of a polymerizable monomer having a phenyl group and/or an anionic polymerizable monomer.

The polymerizable monomer having the phenyl group is not particularly limited, and examples thereof include styrene, divinylbenzene, ethylstyrene, α-methylstyrene, p-chlorostyrene, chloromethylstyrene, and the like. Each of these may be used alone, or two or more kinds of these may be used in combination.

The anionic polymerizable monomer is not particularly limited, and examples thereof may include styrene sulfonate, (meth)acrylic acid, divinylbenzene sulfonate, ethylstyrene sulfonate, α-methylsulfonate, and the like. The salts in this case are not particularly limited, and examples thereof include a sodium salt, a potassium salt, a lithium salt, an ammonium salt, and the like. Each of these may be used alone, or two or more kinds of these may be used in combination. Out of these, styrene sulfonate and/or (meth) acrylic acid is desirable. By including styrene sulfonate and/or (meth)acrylic acid, an obtained insoluble carrier itself has a good level of emulsifiability.

The insoluble carrier obtained by including the anionic polymerizable monomer has a surface charge, and therefore, the insoluble carrier is dispersed well in a solution even without an emulsifier. Here, in the case that an emulsifier is included in suspension of the insoluble carrier and a lipid antigen is supported thereon to prepare a reagent for assaying anti-phospholipid antibodies, the emulsifier may prevent an agglutination of polymer spheres caused by a specific antigen-antibody reaction. In some cases, the emulsifier may be involved in a nonspecific reaction. Therefore, it is desirable not to include an emulsifier in suspension of the insoluble carrier.

In the case that an insoluble carrier including the polymer of the polymerizable monomer having the phenyl group and the anionic polymerizable monomer is used, contents of copolymer components of the respective polymerizable monomers are not particularly limited, however, a copolymer component of the anionic polymerizable monomer is desirably 50 parts by weight or less with respect to 100 parts by weight of a copolymer component of the polymerizable monomer having the phenyl group. In the case that the copolymer component of the anionic polymerizable monomer is more than 50 parts by weight, an obtained insoluble carrier may be less dispersive. The more desirable copolymer component of the anionic polymerizable monomer is 30 parts by weight or less.

The insoluble carrier has a surface charge and the surface charge includes a surface charge generated by the anionic polymerizable monomer, which is the above-described copolymer component, and a surface charge generated by an anion of a piece of a polymerization initiator used in polymerization. An example of the surface charge generated by an anion of a piece of a polymerization initiator can be described as following: in the case that a persulfate such as potassium persulfate is used, a sulfate radical (-OSO₃⁻), a piece, is present on a surface of a copolymer particle, and the sulfate radical is gradually hydrolyzed to be changed as shown by the below formula.

-SO₃⁻ + H₂O → -OH + HOSO₃⁻

The insoluble carrier desirably has a surface charge density of 0.01 to 0.4 µmol/m² in terms of a dissociation concentration of anions in a state of suspension. Here, a medium of the suspension is a medium used in an immunoassay test, and examples thereof include water, saline, serum, and the like.

In the case that the surface charge density is less than 0.01 µmol/m², repulsion between the particles is weak, so that the emulsifiability of the insoluble carrier may be deteriorated. In contrast, in the case that the surface charge density is more than 0.4 µmol/m², electrical repulsion between the insoluble carriers becomes stronger so that autoagglutination is not caused and the insoluble carriers are stable, however, the agglutination caused by the antigen-antibody reaction is also prevented and a highly sensitive measurement may fail to be done.

Further, the insoluble carrier is desirably a sphere having a particle diameter of 0.1 to 0.7 µm. In the case that the particle diameter is less than 0.1 µm, an optical change caused by the agglutination is small, so that a high sensitivity required for a measurement may not be obtained. In contrast, in the case that the particle diameter is more than 0.7 µm, an amount of optical change caused by the particle agglutination exceeds a measurable range so that a measuring range for the assay may become smaller. The more desirable lower limit is 0.2 µm and the more desirable upper limit is 0.5 µm.

A method for polymerizing the polymerizable monomers is not particularly limited, and examples thereof include a method for conducting, by using a polymerization initiator, emulsion polymerization, suspension polymerization, seed polymerization, dispersion polymerization, or the like.

The polymerization initiator is not particularly limited, and examples thereof include persulfate such as potassium persulfate and the like.

A method for supporting the phospholipid antigen on the insoluble carrier is not particularly limited, and examples thereof include a method of supporting by physical bond and/or chemical bond through a conventionally known method.

The reagent for assaying anti-phospholipid antibodies may be used as a one-component type latex reagent by dispersing and dissolving the insoluble carrier supporting the phospholipid antigen thereon and the copolymer in the same medium, or alternatively, it may also be used as a two-component type reagent including a first reagent containing the insoluble carrier supporting the phospholipid antigen thereon and a second reagent including a buffer prepared by adding the copolymer to a medium.

The medium is not particularly limited, and examples thereof include a phosphate buffer, a glycine buffer, a Tris-HCL buffer, a Good's buffer and the like.

Further, a pH of the medium is not particularly limited, however, the desirable lower limit is 5.5 and the desirable upper limit is 8.5, and furthermore, the more desirable lower limit is 6.5.

In the one-component type latex reagent, bovine serum albumin, sucrose, sodium chloride, EDTA·2Na, surfactant and the like may be further dissolved as appropriate.

Furthermore, also in the case that the reagent is used as the two-component type reagent including the latex reagent and the solution reagent, bovine serum albumin, sucrose, sodium chloride, EDTA·2Na, surfactant and the like may be dissolved in the respective reagents as appropriate.

Moreover, a concentration of the bovine serum albumin is not particularly limited, however, the desirable lower limit is 0.1% and the desirable upper limit is 15%, and further, the more desirable lower limit is 1.0% and the more desirable upper limit is 10.0%.

By using the reagent for assaying anti-phospholipid antibodies, a degree of the agglutination caused by the antigen-antibody reaction with the anti-phospholipid antibody in a sample is to be optically measured. Thus, the anti-phospholipid antibody in the sample can be assayed.

As a method for measuring the degree of the agglutination optically, a conventionally known method is used. For example, increase and decrease of scattering light intensity, absorbance and transmission intensity can be measured by changing the size of particles of the insoluble carrier to be used, the concentration of the insoluble carrier, or setting of the reaction time. Further, it is possible to use these methods in combination. Here, a wavelength of light in conducting the measurement is desirably 300 to 900 nm.

As an apparatus used for the optical measurement, an optical device capable of detecting the scattering light intensity, transmission intensity, absorbance and the like can be employed, and any of generally used automatic biochemical analyzers can be used.

By having the reagent for assaying anti-Treponema pallidum antibodies contain a copolymer having a segment derived from 2-methacryloyloxyethyl phosphorylcholine and having a segment derived from a hydrophilic monomer as a sensitizer, it becomes possible to prevent the serum interference in the assay of the anti-Treponema pallidum antibody in serum, so that the anti-Treponema pallidum antibody can be assayed with accuracy. Thus, the inventors of the present invention have completed the assaying anti-Treponema pallidum antibodies of the present invention.

The reagent for assaying anti-Treponema pallidum antibodies contains an insoluble carrier supporting an antigen to syphilis thereon, and a copolymer having a segment derived from 2-methacryloyloxyethyl phosphorylcholine and having a segment derived from a hydrophilic monomer(hereinafter, also referred to simply as copolymer).

The following general formula (1) indicates a structure of the copolymer in the case that methacrylic acid is used as the hydrophilic monomer.

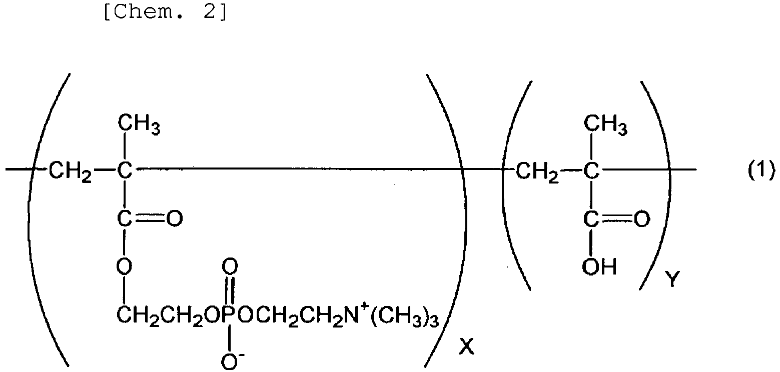

In the reagent for assaying anti-Treponema pallidum antibodies, the copolymer contained therein prevents the serum interference, even in the case that a serum is used as a sample. Therefore, the accurate assaying result can be obtained.

Here, as a method for examining the serum interference, for example, a spike recovery test can be used. The spike recovery test is conducted as follows: a reference material containing an antigen or an antibody, which is a to-be-measured material, at a high concentration is added to a saline (a model of the serum without a variable component contained therein) so that the concentration thereof becomes approximately 0.1 to 5%; a difference between the measured values before and after the addition of the reference material is calculated; the reference material is added to the serum containing an antigen or an antibody, which is a to-be-measured material, and a difference between the measured values before and after the addition of the reference material is calculated in the same manner; defining that the difference of the measured values in the case of adding the reference material to the saline is 100%, a ratio of the difference of the measured values in the case of adding the reference material to the serum is calculated as a recovery ratio; thus, an occurrence of serum interference is examined.

The 2-methacryloyloxyethyl phosphorylcholine is, since a methacryloyl group is included, capable of copolymerizing with another polymerizable monomer. Examples of a copolymerizable hydrophilic monomer include a (meth)acrylic acid monomer, such as 2-hydroxyethyl methacrylate, (meth)acrylate, (meth)acrylamide, and the like.

The hydrophilic monomer is not particularly limited, and examples thereof include (meth) acrylic acid, (meth) acrylamide, and the like. Out of these, (meth)acrylic acid, which is cationic, is suitable, because (meth)acrylic acid is expected to repulse electrostatically from protein and the like in components in blood.

Here, (meth)acrylic acid indicates acrylic acid or methacrylic acid.

A ratio between the segment derived from 2-methacryloyloxyethyl phosphorylcholine and a segment derived from a hydrophilic monomer in the copolymer is not particularly limited, and can be selected properly as needed. However, it is desirable to be 5: 5 to 3:7 in a molar ratio. In the case that the ratio of the segment derived from 2-methacryloyloxyethyl phosphorylcholine to the segment derived from a hydrophilic monomer is less than this range, an occurrence of serum interference during the assay of the anti-Treponema pallidum antibody may not be prevented efficiently.

A weight-average molecular weight of the copolymer is not particularly limited, however, the desirable lower limit is 50000 and the desirable upper limit is 5 million. In the case that the weight-average molecular weight is less than 50000, an effect of accelerating agglutination may be lost. In contrast, in the case that the weight-average molecular weight is more than 5 million, reproducibility and the like may be deteriorated since viscosity of the reagent becomes too high in the case of adding the copolymer to the reagent.

With regard to a content of the copolymer in the reagent for assaying anti-Treponema pallidum antibodies, the desirable lower limit is 0.1 1 (w/v)% and the desirable upper limit is 1.2 (w/v)%. In the case that the content is less than 0.1 1 (w/v)%, the occurrence of serum interference may not be prevented efficiently. Further, in the case that the content is more than 1.2 (w/v)%, the reproducibility may be deteriorated, since the viscosity of the reagent becomes too high.

The more desirable lower limit is 0.2 (w/v)% and the more desirable upper limit is 0.8 (w/v)%.

The reagent for assaying anti-Treponema pallidum antibodies contains an insoluble carrier supporting the Treponema pallidum antigen thereon, in addition to the copolymer.

As the Treponema pallidum antigen, for example, an antigen derived from a bacterial cell of a Treponema pallidum is desirable.

The insoluble carrier is not particularly limited, and examples thereof include an insoluble carrier comprising polystyrene, styrene-styrenesulfonate polymer, acrylonitrile-butadiene-styrene copolymer, vinylchloride-acrylate copolymer, polyvinyl acetate acrylate or the like.

With regard to the particle diameter of the insoluble carrier, though depending on a measuring method or a measuring apparatus to be used, the desirable lower limit is 0.05 µm and the desirable upper limit is 1.0 µm. In the case that the particle diameter is less than 0.05 µm, an optical change caused by the agglutination may be small, so that a high sensitivity required for the measurement may not be obtained. In contrast, in the case that the particle diameter is more than 1.0 µm, the optical change caused by the particle agglutination exceeds a measurable range, so that a measuring range for the assay may become smaller.

A method for supporting the Treponema pallidum antigen on the insoluble carrier is not particularly limited, and examples thereof include a method for supporting by physical bond or chemical bond through a conventionally known method. The Treponema pallidum antigen used here may be crushed bacterial cells or purified bacterial cells. Further, one or more kinds of an antigen artificially composed by genetic engineering may be used in combination.

The reagent for assaying anti-Treponema pallidum antibodies may be used as a one-component type latex reagent by dispersing and dissolving the insoluble carrier supporting the Treponema pallidum antigen thereon and the copolymer in the same medium, or alternatively, it may also be used as a two-component type reagent including a first reagent containing the insoluble carrier supporting the Treponema pallidum antigen thereon and a second reagent including a buffer prepared by adding the copolymer to a medium.

The medium is not particularly limited, and examples thereof include a phosphate buffer, a glycine buffer, a Tris-HCL buffer and the like.

In the one-component type latex reagent, bovine serum albumin, sucrose, sodium chloride, EDTA·2Na, surfactant and the like may be dissolved as appropriate.

Furthermore, also in the case that the reagent is used as the two-component type reagent, bovine serum albumin, sucrose, sodium chloride, EDTA·2Na, surfactant and the like may be dissolved in the respective reagents as appropriate.

Moreover, a concentration of the bovine serum albumin is not particularly limited, however, the desirable lower limit is 0.1% and the desirable upper limit is 15%, and further, the more desirable lower limit is 1.0% and the more desirable upper limit is 10.0%.

By using the reagent for assaying anti-Treponema pallidum antibodies, a degree of the agglutination caused by the antigen-antibody reaction with an anti-Treponema pallidum antibody in a sample is to be optically measured or visually observed. Thus, the anti-Treponema pallidum antibody in the sample can be assayed.

The desirable lower limit of a pH when the antigen-antibody reaction is carried out by using the reagent for assaying the anti-Treponema pallidum antibodies is 4.5 and the desirable upper limit is 10.0, and further, the more desirable lower limit is 6.0 and the more desirable upper limit is 8.0.

Moreover, the desirable lower limit of a reaction temperature is 0°C and the desirable upper limit is 50°C. A reaction time is selected as appropriate.

As a method for measuring the degree of the agglutination optically, a conventionally known method is used. For example, increase and decrease of scattering light intensity, absorbance and transmission intensity can be measured by changing the size of particles of the insoluble carrier to be used, the concentration of the insoluble carrier, or setting of the reaction time. Further, it is possible to use these methods in combination. Here, a wavelength of light in conducting the measurement is desirably 300 to 900 nm.

As an apparatus used for the optical measurement, an optical device capable of detecting the scattering light intensity, transmission intensity, absorbance and the like can be employed, and any of generally used automatic biochemical analyzers can be used.

As a method for visually observing the degree of the agglutination, normally, a method for mixing a sample and the reagent for assaying anti-Treponema pallidum antibodies on a test slide and for determining a presence or absence of agglutination after vibrating the liquid mixture, and the like can be used. Here, to observe the degree of the agglutination, a method for filming an agglutination state with a video camera and for processing the image can also be used, in addition to the visual observation.

According to the present disclosure, it is possible to provide assaying anti-phospholipid antibodies, which enables an accurate diagnosis of syphilitic infection and is excellent in long-term storage stability, and assaying anti-Treponema pallidum antibodies, which enables the accurate diagnosis of syphilitic infection by preventing an occurrence of serum interference.

### (Example 1)

### (1) Preparation of a reagent for assaying anti-phospholipid antibodies

According to the following procedures, a two-component type reagent including a first reagent and a second reagent was prepared.

### (1-1) Preparation of first reagent

An amount of 1.2% by weight of LipidureD02 (made by NOF Corp., molecular weight of 550000) neutralized with NaOH was added to a phosphate buffer containing 1% of a bovine serum albumin ("Lot A", made by Serologicals Corporation) so as to prepare the first reagent.

### (1-2) Preparation of second reagent

A latex liquid of MediAce RPR (made by Sekisui Chemical Co., Ltd.) was used as it was as the second reagent. The latex liquid was prepared by sensitizing a lipid antigen including cardiolipin, phosphatidylcholine and cholesterol to a polystyrene latex having the average particle diameter of 0.400 µm and having a sulfone group of 0.38 µmol/m².

### (Example 2)

The reagent for assaying anti-phospholipid antibodies was prepared in the same manner as in Example 1, except that, in preparation of the first reagent (1-1), 0.70% by weight of LipidureD03 (made by NOF Corp., molecular weight of 1 million) neutralized with NaOH was added to a phosphate buffer containing 1% of bovine serum albumin ("Lot A", made by Serologicals Corporation) so as to prepare the first reagent.

### (Example 3)

The reagent for assaying anti-phospholipid antibodies was prepared in the same manner as in Example 1, except that, in preparation of the first reagent (1-1), 0.45% by weight of LipidureD05 (made by NOF Corp., molecular weight of 1 million) neutralized with NaOH was added to a phosphate buffer containing 1% of bovine serum albumin ("Lot A", made by Serologicals Corporation) so as to prepare the first reagent.

### (Comparative Example 1)

The reagent for assaying anti-phospholipid antibodies was prepared in the same manner as in Example 1, except that, in preparation of the first reagent (1-1), 1.2% by weight of pullulan (made by Hayashibara Co., Ltd.), in stead of LipidureD02, was added to a phosphate buffer containing 1% of bovine serum albumin ("Lot A", made by Serologicals Corporation) and dissolved therein.

### (Comparative Example 2)

The reagent for assaying anti-phospholipid antibodies was prepared in the same manner as in Example 1, except that, in preparation of the first reagent (1-1), 1.0% by weight of polyvinylpyrrolidone, in stead of LipidureD02, was added to a phosphate buffer containing 1% of bovine serum albumin ("Lot A", made by Serologicals Corporation) and dissolved therein.

### (Comparative Example 3)

The reagent for assaying anti-phospholipid antibodies was prepared in the same manner as in Example 1, except that, in preparation of the first reagent (1-1), 2.0% by weight of dextran (molecular weight of 500000: made by Sigma Co.), in stead of LipidureD02, was added to a phosphate buffer containing 1% of bovine serum albumin ("LotA", made by Serologicals Corporation) and dissolved therein.

### (Evaluation (1))

With respect to each of the reagents for assaying anti-phospholipid antibodies obtained in Examples 1 to 3 and Comparative Examples 1 to 3, the following evaluations were conducted.

### (1) Measurement of amount of absorbance change immediately after preparation

By using each of RPR reference serums (made by Sekisui Chemical Co., Ltd.) having five different concentrations of 0.0, 1.0, 2.0, 4.0 and 8.0 R.U. as a reference solution of the anti-phospholipid antibody, the following measurement was carried out.

An amount of 20 µL of RPR reference serum was sampled as the reference solution of the anti-phospholipid antibody, and then, 180 µL of the first reagent was mixed thereto and maintained at 37°C for an appropriate period of time. Thereafter, 60 µL of the second reagent was further added and stirred. Then, the amount of the change of the absorbance in a period between about 80 seconds and 300 seconds at a wavelength of 700 nm was measured to obtain the amount of the absorbance change (Δabs). For measuring the absorbance, Hitachi 7170 autoanalyzer was used. Here, R.U. is a unit indicating an antibody titer of the anti-phospholipid antibody derived from the syphilitic infection in the case that the serum is measured by using MediAce RPR (made by Sekisui Chemical Co., Ltd.), which is a reagent for assaying the anti-phospholipid antibodies.

The results were shown in Table 1.

**[Table 1]**

| | | Example 1 | Example 2 | Example 3 | Comparative Example 1 | Comparative Example 2 | Comparative Example 3 |
|---|---|---|---|---|---|---|---|
| Sensitizer | | LipidureD02 | LipidureD03 | LipidureD05 | Pullulan | PVP | Dextran |
| Amount of absorbance change | 0.0 R.U. | 0 | 0 | 0 | 0 | 0 | 0 |
| | 1.0 R.U. | 420 | 537 | 367 | 569 | 389 | 527 |
| | | 995 | 1088 | 872 | 994 | 867 | 1045 |
| | 4.0 R.U. | 2264 | 2582 | 2152 | 2315 | 2359 | 2084 |
| | 8.0 R.U. | 4056 | 4239 | 3770 | 4012 | 4307 | 3293 |

| | | | | | | | |
|---|---|---|---|---|---|---|---|
| Unit: Δ abs | | | | | | | |

### (2) Evaluation on storage stability

After storage of the first reagent at a temperature of 30°C, measurement of the reference solution of the anti-phospolipid antibody was carried out as described in the measurement (1) to obtain a reduction ratio of Δabs in the case that Δabs immediately after the preparation was defined as 100%; thus, the storage stability was evaluated. In general, reagents for assaying anti-phospholipid antibodies are stored at a temperature of 2 to 10°C, however, storage thereof at a temperature of 30°C allows evaluation of the storage stability as an accelerated test.

Here, the measurement was conducted after a lapse of 5 days, 15 days, 18days and 25 days from the preparation.

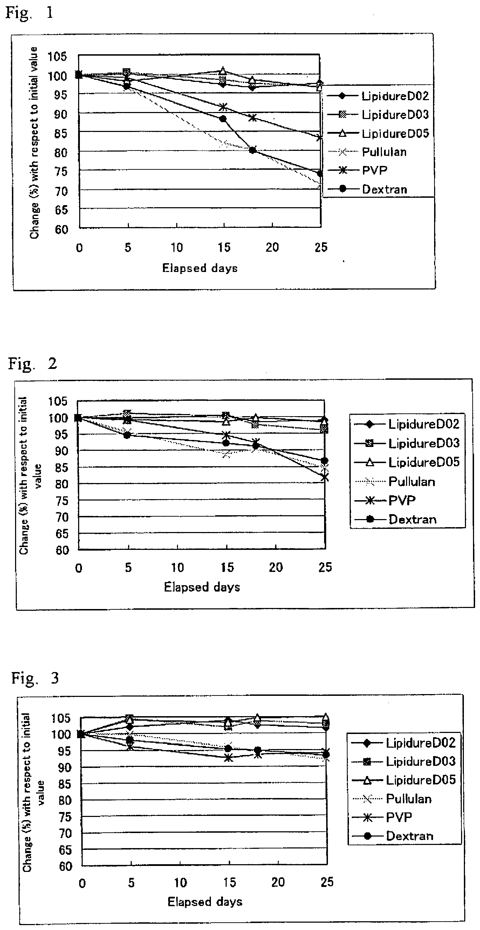

The results obtained by using each one of the reference solutions of the anti-phospholipid antibody of 1.0, 2.0, 4.0 and 8.0 R.U. were respectively shown in Fig. 1 to Fig. 4.

Use of either of the reference solutions of the anti-phospholipid antibody with a concentration of 1.0 R.U. or 2.0 R.U., as shown in Fig. 1 and Fig. 2, caused reductions of 15 to 30% in the amounts of absorbance change after a lapse of 25 days in cases where pullulan, PVP and dextran were used. In contrast, in cases where LipidureD02, LipidureD03 and LipidureD05 were used, the amounts of absorbance change were found to be 5% or less.

### (Example 4)

The reagent for assaying anti-phospholipid antibodies was prepared in the same manner as in Example 1, except that, in preparation of the first reagent (1-1), 0.66% by weight of LipidureD03 (made by NOF Corp.) neutralized with NaOH was added to a phosphate buffer containing 1% of bovine serum albumin (Lot B).

### (Example 5)

The reagent for assaying anti-phospholipid antibodies was prepared in the same manner as in Example 1, except that, in preparation of the first reagent (1-1), 0.66% by weight of LipidureD03 (made by NOF Corp.) neutralized with NaOH was added to a phosphate buffer containing 1% of bovine serum albumin (Lot C) .

### (Example 6)

The reagent for assaying anti-phospholipid antibodies was prepared in the same manner as in Example 1, except that, in preparation of the first reagent (1-1), 0.66% by weight of LipidureD03 (made by NOF Corp.) neutralized with NaOH was added to a phosphate buffer containing 1% of bovine serum albumin (Lot D).

### (Comparative Example 4)

The reagent for assaying anti-phospholipid antibodies was prepared in the same manner as in Example 1, except that, in preparation of the first reagent (1-1), 1.1% by weight of pullulan (made by Hayashibara Co., Ltd.), in stead of LipidureD02, was added to a phosphate buffer containing 1% of bovine serum albumin (Lot B) and dissolved therein.

### (Comparative Example 5)

The reagent for assaying anti-phospholipid antibodies was prepared in the same manner as in Example 1, except that, in preparation of the first reagent (1-1), 1.1% by weight of pullulan (made by Hayashibara Co., Ltd.), in stead of LipidureD02, was added to a phosphate buffer containing 1% of bovine serum albumin (Lot C) and dissolved therein.

### (Comparative Example 6)

The reagent for assaying anti-phospholipid antibodies was prepared in the same manner as in Example 1, except that, in preparation of the first reagent (1-1), 1.1% by weight of pullulan (made by Hayashibara Co., Ltd.), in stead of LipidureD02, was added to a phosphate buffer containing 1% of bovine serum albumin (Lot D) and dissolved therein.

### (Evaluation (2))

With respect to each of the reagents for assaying anti-phospholipid antibodies obtained in Examples 4 to 6 and Comparative Examples 4 to 6, the following evaluations were conducted.

### (1) Measurement of amount of absorbance change immediately after preparation

By using each of RPR reference serums (made by Sekisui Chemical Co., Ltd.) having five different concentrations of 0.0, 1.0, 2.0, 4.0 and 8.0 R.U. as a reference solution of the anti-phospholipid antibody, the following measurement was carried out.

An amount of 20 µL of RPR reference serum was sampled as the reference solution of the anti-phospholipid antibody, and then, 180 µL of the first reagent was mixed thereto and maintained at 37°C for an appropriate period of time. Thereafter, 60 µL of the second reagent was further added and stirred. Then, the amount of the change of the absorbance in a period between about 80 seconds and 300 seconds at a wavelength of 700 nm was measured to obtain the amount of the absorbance change (Δabs). For measuring the absorbance, Hitachi 7170 autoanalyzer was used. The results were shown in Table 2.

**[Table 2]**

| | | Example 4 | Example 5 | Example 6 | Comparative Example 4 | Comparative Example 5 | Comparative Example 6 |
|---|---|---|---|---|---|---|---|
| BSA Lot | | B | C | D | B | C | D |
| Sensitizer | | LipidureD03 | LipidureD03 | LipidureD03 | Pullulan | Pullulan | Pullulan |
| Amount of absorbance change | 0.0 R.U. | 0 | 0 | 0 | 0 | 0 | 0 |
| | 1.0 R.U. | 281 | 353 | 417 | 207 | 295 | 329 |
| | 2.0 R.U. | 759 | 931 | 1043 | 559 | 723 | 793 |
| | 4.0 R.U. | 2187 | 2432 | 2589 | 1611 | 1926 | 2058 |
| | 8.0 R.U. | 4042 | 4276 | 4392 | 3445 | 3797 | 3897 |

| | | | | | | | |
|---|---|---|---|---|---|---|---|
| Unit: Δ abs | | | | | | | |

### (2) Evaluation on storage stability

After storage of the first reagent at a temperature of 30°C, measurement of the reference solution of the anti-phospolipid antibody was carried out as described in the measurement (1) to obtain a reduction ratio of Δabs in the case that Δabs immediately after the preparation was defined as 100%; thus, the storage stability was evaluated. In general, the reagent for assaying anti-phospholipid antibodies are stored at a temperature of 2 to 10°C, however, storage thereof at a temperature of 30°C allows evaluation of the storage stability as an accelerated test.

Here, the measurement was conducted after a lapse of 7 days and 14 days from the preparation.

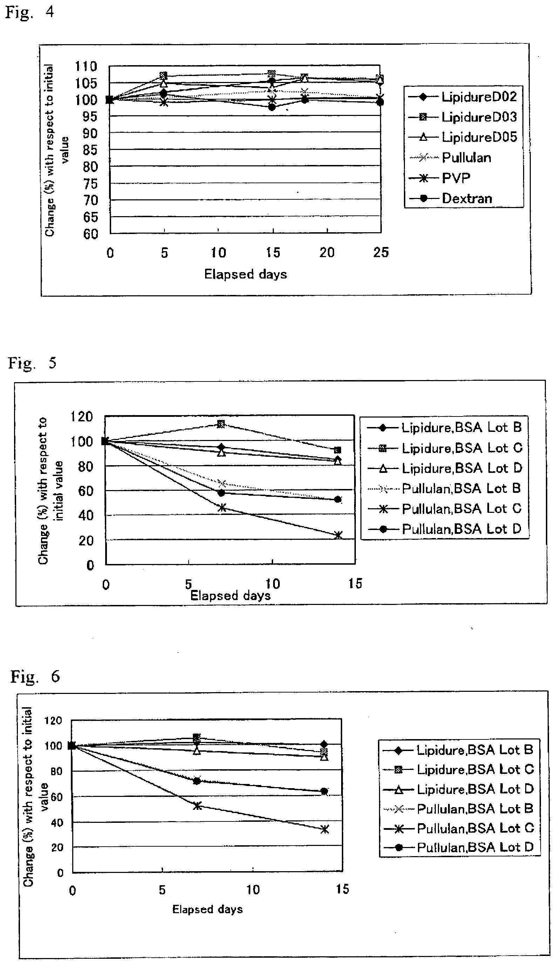

The results obtained by using each one of the reference solutions of the anti-phospholipid antibody of 1.0 and 2.0 R.U. were respectively shown in Fig. 5 and 6.

As shown in Fig. 5 and 6, in the case that Lot D of BSA and pullulan were used in combination, a great loss in sensitivity was observed after the storage at a temperature of 30°C for 14 days. However, in the case that Lot D of BSA and LipidureD03 were used in combination, only a small loss in sensitivity was observed. Accordingly, the result shows that the storage stability was less likely to be affected by BSA.

### (Example 7)

### (1) Preparation of reagent for assaying anti-Treponema pallidum antibodies

According to the following procedures, a two-component type reagent formed by a first reagent including a Treponema pallidum antigen-supporting latex solution and a second reagent including a sample diluent was prepared.

### (1-1) Preparation of Treponema pallidum antigen-supporting latex solution

An amount of 400 µl of Treponema pallidum antigen solution dissolved in a 100 mM phosphate buffer (pH 7.4) to have a protein concentration of 150 µg/ml was added to 100 µl of polystylene latex liquid (10(w/v)% of solid content, made by Sekisui Chemical Co., Ltd.) having an average particle diameter of 0.400 µm, and stirred at a temperature of 4°C for 1 hour. Subsequently, 2 ml of a 100 mM phosphate buffer (pH 7.4) containing 1 (w/v) of bovine serum albumin (made by Serologicals Corporation; BSA, Lot 1) was added thereto and stirred for 1.5 hours.

The resulting liquid was centrifuged at 18000 rpm at a temperature of 10°C for 30 minutes and the obtained precipitate was added to 4 ml of a 100 mM phosphate buffer (pH 7.4) containing 0.25 (w/v)% of BSA (Lot 1) to suspend the latex, so that a Treponema pallidum antigen-supporting latex solution was prepared.

### (1-2) Preparation of sample diluent

An amount of 0.2 (w/v)% of Lipidure (copolymer of 2-methacryloyloxyethyl phosphorylcholine and methacrylic acid, molecular weight of 1 million, made by NOF Corp.) was added to a 100 mM phosphate buffer (pH 7.4) containing 1 (w/v)% of BSA (Lot 1).

### (Example 8)

The reagent for assaying anti-Treponema pallidum antibodies was prepared in the same manner as in Example 7, except that 5 ml of a 100 mM phosphate buffer (pH 7.4) containing 0.25 (w/v)% of BSA (Lot 1) was added to the obtained precipitate in preparation of a Treponema pallidum antigen-supporting latex solution (1-1) in Example 7, and 0.6 (w/v)% of Lipidure was added to the 100 mM phosphate buffer (pH7.4) in preparation of a sample diluent (1-2).

### (Example 9)

The reagent for assaying anti-Treponema pallidum antibodies was prepared in the same manner as in Example 7, except that 12 ml of a 100 mM phosphate buffer (pH 7.4) containing 0.25 (w/v) % of BSA (Lot 1) was added to the obtained precipitate, in preparation of a Treponema pallidum antigen-supporting latex solution (1-1) in Example 7, and 1.0 (w/v)% of Lipidure was added to the 100 mM phosphate buffer in preparation of a sample diluent (1-2).

### (Comparative Example 7)

The reagent for assaying anti-Treponema pallidum antibodies was prepared in the same manner as in Example 7, except that 1 (w/v)% of pGEMA (homopolymer of glycosyl ethyl methacrylate, average molecular weight of 1.14 million, made by Nippon Fine Chemical Co., Ltd.), instead of Lipidure, was added to the 100 mM phosphate buffer (pH 7.4) containing 1% of BSA(Lot1) in preparation of a sample diluent (1-2) in Example 7.

### (Evaluation (3))

### (1) Measurement of reference solution of anti-Treponema pallidum antibody

By using each of syphilis-positive reference serums (made by Sekisui Chemical Co., Ltd.) having five different concentrations as a reference solution of the anti-phospholipid antibody, the following measurement was carried out.

An amount of 16 µL of syphilis-positive reference serum was sampled as the reference solution of the anti-Treponema pallidum antibody. An amount of 175 µL of the sample diluent was mixed thereto and maintained at a temperature of 37°C for an appropriate period of time, and thereafter, 25 µL of the Treponema pallidum antigen-supporting latex solution was further added and stirred. Then, the amount of the change of the absorbance in a period between about 80 seconds and 300 seconds at a wavelength of 700 nm was measured to obtain the amount of the absorbance change (Δabs). For measuring the absorbance, Hitachi 7170 autoanalyzer was used. The results were shown in Table 3. Here, T.U. in Table 3 is a unit indicating an antibody titer of the anti-Treponema pallidum antibody in the case that the serum is measured by using MediAce TPLA (made by Sekisui Chemical Co., Ltd.), which is the reagent for assaying anti-Treponema pallidum antibodies. The value of 10 T.U. or more is regarded as positive.

**[Table 3]**

| | | Example 7 | Example 8 | Example 9 | Comparative Example 7 |
|---|---|---|---|---|---|
| Amount of absorbance change (Δ abs) | 0.T.U. | -48 | -5 | 46 | 11 |
| | 18 T.U. | 1 | 166 | 296 | 212 |
| | 37 T.U. | 79 | 423 | 553 | 552 |
| | 119 T.U. | 384 | 1611 | 931 | 1884 |
| | 234 T.U. | 817 | 2736 | 985 | 2874 |

### (2) Spike recovery test

An amount of 5 µL of the reference solution of the anti-Treponema pallidum antibody of 2000 T.U. was added to 245 µL of saline. Then, a difference of antibody titers measured before and after the addition was obtained by measuring amounts of the absorbance changes before and after the addition in the same manner as in evaluation (1). In the same manner, 5 µL of the reference solution of the anti-Treponema pallidum antibody of 2000 T.U. were respectively added to 245 µL of the reference solutions in five variations and the difference of the antibody titers measured before and after the addition was obtained. Thereafter, the recovery ratio was calculated, defining that the difference of the antibody titers measured before and after the addition in the case that the reference solution of the anti-Treponema pallidum antibody was added to the saline as 100%. The results were shown in Table 4.

**[Table 4]**

| | | Example 7 | Example 8 | Example 9 | Comparative Example 7 |
|---|---|---|---|---|---|
| Recovery ratio (%) | Saline | 100 | 100 | 100 | 100 |
| | Serum 1 | 107 | 90 | 102 | 80 |
| | Serum 2 | 80 | 92 | 92 | 78 |
| | Serum 3 | 93 | 98 | 107 | 83 |
| | Serum 4 | 88 | 97 | 72 | 69 |
| | Serum 5 | 107 | 98 | 84 | 81 |

As shown in Table 4, the recovery ratio was significantly improved in each of Examples 7 to 9, in which Lipidure was used as a reaction accelerator, compared to Comparative Example 7, in which pGEMA was used as the reaction accelerator.

### (Example 10)

A sample diluent was prepared in the same manner as in Example 7, except that BSA (Lot 2) was used in preparation of the sample diluent (1-2) in Example 7.

### (Example 11)

A sample diluent was prepared in the same manner as in Example 7, except that BSA (Lot 3) was used in preparation of the sample diluent (1-2) in Example 7.

### (Example 12)

A sample diluent was prepared in the same manner as in Example 7, except that BSA (Lot 4) was used in preparation of the sample diluent (1-2) in Example 7.

### (Comparative Example 8)

A sample diluent was prepared in the same manner as in Comparative Example 7, except that BSA (Lot 2) was used in preparation of the sample diluent (1-2) in Comparative Example 7.

### (Comparative Example 9)

A sample diluent was prepared in the same manner as in Comparative Example 7, except that BSA (Lot 3) was used in preparation of the sample diluent (1-2) in Comparative Example 7.

### (Comparative Example 10)

A sample diluent was prepared in the same manner as in Comparative Example 7, except that BSA (Lot 4) was used in preparation of the sample diluent (1-2) in Comparative Example 7.

### (Comparative Example 11)

The reagent for assaying anti-Treponema pallidum antibodies was prepared in the same manner as in Example 7, except that 1 (w/v)% of pullulan (made by Hayashibara Co., Ltd.), instead of Lipidure, was added to the 100 mM phosphate buffer (pH 7.4) containing 1% of BSA(Lot 1) in preparation of a sample diluent (1-2) in Example 7.

### (Comparative Example 12)

A sample diluent was prepared in the same manner as in Comparative Example 11, except that BSA (Lot 2) was used in preparation of the sample diluent (1-2) in Comparative Example 11.

### (Comparative Example 13)

A sample diluent was prepared in the same manner as in Comparative Example 11, except that BSA (Lot 3) was used in preparation of the sample diluent (1-2) in Comparative Example 11.

### (Comparative Example 14)

The reagent for assaying anti-Treponema pallidum antibodies was prepared in the same manner as in Example 7, except that 0.1 (w/v)% of sodium alginate (made by Nacalai Tesque, Inc.), instead of Lipidure, was added to the 100 mM phosphate buffer (pH 7.4) containing 1% of BSA(Lot 2), instead of BSA(Lot 1), in preparation of a sample diluent (1-2) in Example 7.

### (Comparative Example 15)

A sample diluent was prepared in the same manner as in Comparative Example 14, except that BSA (Lot 3) was used in preparation of the sample diluent (1-2) in Comparative Example 14.

### (Comparative Example 16)

A sample diluent was prepared in the same manner as in Comparative Example 14, except that BSA (Lot 4) was used in preparation of the sample diluent (1-2) in Comparative Example 14.

### (Evaluation (4))

### (1) Storage stability test

With regard to the reagent for assaying anti-Treponema pallidum antibodies obtained in each of Examples 7, 10 to 12, and Comparative Examples 7 to 16, a storage stability test was conducted.

Immediately after the preparation of the reagent and after storage of the sample diluent at a temperature of 30°C, storage stabilities were measured by measuring 37 T.U. of the syphilis-positive reference serum in the same manner as the method in the procedure (1) of Evaluation (3) so as to obtain the amount of the absorbance change in the case that the absorbance change immediately after the preparation was defined as 100%. In general, reagents for assaying anti-Treponema pallidum antibodies are stored at a temperature of 2 to 10°C, however, storage thereof at a temperature of 30°C allows evaluation of the storage stability as an accelerated test.

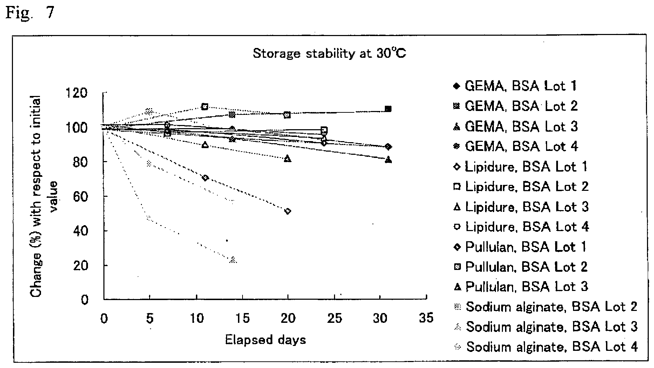

Here, with regard to each of Examples 7, and 10 to 12, measurements were conducted after a lapse of 7 days and 24 days from the preparation. Further, the measurements were conducted: after a lapse of 14 days and 31 days from preparation, with regard to each of Comparative Examples 7 to 10; after a lapse of 11 days and 20 days from the preparation, with regard to each of Comparative Examples 11 to 13; and after a lapse of 5 days and 14 days from the preparation, with regard to Comparative Examples 14 to 16. The results were shown in Fig. 7.

As shown in Fig. 7, in the case that Lipidure was used as a reaction accelerator, the storage stability was excellent regardless of a lot-to-lot variation of BSA. However, in the case of using another reaction accelerator, the lot-to-lot variation of BSA caused the reagent with significantly poor storage stability in some cases. Accordingly, in the case that Lipidure is used as the reaction accelerator, it becomes possible to provide a reagent for assaying anti-Treponema pallidum antibodies, which allows highly accurate assay and also have excellent storage stability.

### BRIEF DESCRIPTION OF THE DRAWINGS

Fig. 1 is a graph illustrating a transition of the amounts of the absorbance change in Examples 1 to 3 and in Comparative Examples 1 to 3, in which the reference solutions of the anti-phospholipid antibody of 1.0 R.U. are used.
Fig. 2 is a graph illustrating a transition of the amounts of the absorbance change in Examples 1 to 3 and in Comparative Examples 1 to 3, in which the reference solutions of the anti-phospholipid antibody of 2.0 R.U. are used.
Fig. 3 is a graph illustrating a transition of the amounts of the absorbance change in Examples 1 to 3 and in Comparative Examples 1 to 3, in which the reference solutions of the anti-phospholipid antibody of 4.0 R.U. are used.
Fig. 4 is a graph illustrating a transition of the amounts of the absorbance change in Examples 1 to 3 and in Comparative Examples 1 to 3, in which the reference solutions of the anti-phospholipid antibody of 8.0 R.U. are used.
Fig. 5 is a graph illustrating a transition of the amounts of the absorbance change in Examples 4 to 6 and in Comparative Examples 4 to 6, in which the reference solutions of the anti-phospholipid antibody of 1.0 R.U. are used.
Fig. 6 is a graph illustrating a transition of the amounts of the absorbance change in Examples 4 to 6 and in Comparative Examples 4 to 6, in which the reference solution of the anti-phospholipid antibody of 2.0 R.U. are used.
Fig. 7 is a graph illustrating a transition of the amounts of the absorbance change in Examples 7 to 10 and in Comparative Examples 7 to 16, in which the reference solutions of the anti-Treponema pallidum antibody of 37 T.U. are used.

## Claims

1. A method for assaying an anti-Treponema pallidum antibody comprising contacting
a reagent comprising a particle supporting a Treponema pallidum antigen thereon and a copolymer dissolving in a medium and obtained by copolymerizing 2-methacryloyloxyethyl phosphorylcholine and a hydrophilic monomer with
a sample taken from a subject
to cause a reaction between an anti-Treponema pallidum antibody in the sample with the Treponema pallidum antigen supported on the particle, and optically measuring a degree of agglutination caused by the reaction to assay the anti-Treponema pallidum antibody in the sample.

2. The method of claim 1,
wherein the Treponema pallidum antigen is an antigen derived from a bacterial cell of a Treponema pallidum.

3. The method of claims 1 or 2,
wherein a content of the copolymer in the reagent for assaying anti-Treponema palladium antibody is 0.1 to 1.2 (w/v) %

4. The method of claims 1, 2 or 3,
wherein the particle is a polymer of a polymerizable monomer comprising a phenyl group and/or a polymer of an anionic polymerizable monomer, and has a surface charge density of 0.01 to 0.4 µmol/m² in terms of a dissociation concentration of anions in a state of suspension and has a spherical shape of 0.1 to 0.7 µm in particle diameter.

## Patentansprüche

1. Verfahren zum Untersuchen eines anti-Treponema pallidum Antikörpers, umfassend Inkontaktbringen
eines Reagenzes, das einen Partikel, der ein Treponema pallidum Antigen darauf trägt, und ein Copolymer, das sich in einem Medium löst, umfasst und durch Copolymerisieren von 2-Methacryloyloxyethylphosphorylcholin und einem hydrophilen Monomer erhalten ist, mit
einer Probe, die einem Subjekt entnommen wurde,
um eine Reaktion zwischen einem anti-Treponema pallidum Antikörper in der Probe mit dem auf dem Partikel getragenen Treponema pallidum Antigen hervorzurufen und einen Grad einer durch die Reaktion hervorgerufenen Agglutination optisch zu messen, um den anti-Treponema pallidum Antikörper in der Probe zu untersuchen.

2. Verfahren nach Anspruch 1,
wobei das Treponema pallidum Antigen ein Antigen ist, das von einer Zelle des Bakteriums Treponema pallidum abgeleitet ist.

3. Verfahren nach Anspruch 1 oder 2,
wobei ein Gehalt des Copolymers in dem Reagenz zum Untersuchen eines anti-Treponema pallidum Antikörpers 0,1 bis 1,2 % Masse pro Volumen beträgt.

4. Verfahren nach einem der Ansprüche 1, 2 oder 3,
wobei der Partikel ein Polymer eines polymerisierbaren Monomers, das eine Phenylgruppe umfasst, und/oder ein Polymer eines anionischen polymerisierbaren Monomers ist, und eine Oberflächenladungsdichte von 0,01 bis 0,4 µmol/m² im Sinne einer Lösungskonzentration von Anionen in einem Suspensionszustand aufweist und eine sphärische Form von 0,1 bis 0,7 µm im Partikeldurchmesser aufweist.

## Revendications

1. Procédé de dosage d'un anticorps anti-Treponema pallidum comprenant la mise en contact
d'un réactif comprenant une particule supportant un antigène de Treponema pallidum sur son dessus et un copolymère se dissolvant dans un milieu et obtenu par copolymérisation de 2-méthacryloyloxyéthyl-phosphorylcholine et d'un monomère hydrophile avec
un échantillon prélevé chez un sujet
pour occasionner une réaction entre un anticorps anti-Treponema pallidum dans l'échantillon avec l'antigène de Treponema pallidum supporté sur la particule, et la mesure optique d'un degré d'agglutination occasionné par la réaction pour doser l'anticorps anti-Treponema pallidum dans l'échantillon.

2. Procédé selon la revendication 1,
dans lequel l'antigène de Treponema pallidum est un antigène dérivé d'une cellule bactérienne de Treponema pallidum.

3. Procédé selon les revendications 1 ou 2,
dans lequel une teneur du copolymère dans le réactif pour doser l'anticorps anti-Treponema pallidum est de 0,1 à 1,2 % (masse/volume).

4. Procédé selon les revendications 1, 2 ou 3,
dans lequel la particule est un polymère d'un monomère polymérisable comprenant un groupe phényle et/ou un polymère d'un monomère anionique polymérisable, et présente une densité de charge en surface de 0,01 à 0,4 µmol/m² en termes d'une concentration de dissociation d'anions dans un état de suspension et présente une forme sphérique de 0,1 à 0,7 µm en diamètre de particule.
